# EUROPÄISCHE PATENTANMELDUNG

(11) **EP 0 958 800 A2**
(43) Veröffentlichungstag der Anmeldung: **24.11.1999**
(21) Anmeldenummer: 99108744.6
(22) Anmeldetag: 03.05.1999
(51) Int. Cl.: A61F 13/15

(54) **Slipeinlage**

(30) Priorität: 15.05.1998 DE 19821821; 10.12.1998 DE 29821773 U
(71) Anmelder: Bütow, Evelyn, 45711 Datteln (DE)
(72) Erfinder: Bütow, Evelyn, 45711 Datteln (DE)
(74) Vertreter: Rohmann, Michael, Dr.

(57) **Zusammenfassung**

Slipeinlage mit körperseitiger Saugauflage (1), körperabgewandter Klebeschicht (2) und auf der Klebeschicht (2) aufgebrachtem Schutzstreifen (3). Entlang einer von einer Längsseite (4) zur anderen Längsseite (5) der Slipeinlage verlaufenden Diagonalen ist eine Trennfuge (6) in der Saugauflage (1) vorgesehen. Die Saugauflage (1) ist entlang der Diagonalen in zwei Teilsaugauflagen (10, 11) auftrennbar.

## Beschreibung

Die Erfindung betrifft eine Slipeinlage mit körperseitiger Saugauflage, körperabgewandter Klebeschicht und auf der Klebeschicht aufgebrachtem Schutzstreifen. - Derartige Slipeinlagen, insbesondere für Damenslips, sind in verschieden Ausführungsformen bekannt. Die Länge einer solchen Slipeinlage beträgt regelmäßig ein Mehrfaches der Breite. Normalerweise beträgt die Länge etwa das zwei- bis dreifache der Breite der Slipeinlage. In der Regel umfaßt die körperseitige Saugauflage zumindest einen saugfähigen Vliesstoff. Die Klebeschicht bzw. Adhäsionsschicht dient dazu, die Slipeinlage bzw. Saugauflage im Slip festzulegen und gegen ein Verrutschen zu sichern. Die Slipeinlagen werden mit auf der Klebeschicht aufgebrachtem Schutzstreifen auf den Markt gebracht und der Schutzstreifen wird vor der Benutzung der Slipeinlage von der Klebeschicht entfernt.

Die im Handel befindlichen Slipeinlagen weisen in der Regel eine Standardgröße auf und sind von ihrer Größe her für einen großen Teil von Slips bzw. Damenslips geeignet. Aufgrund ihrer Form und Größe sind die herkömmlichen Slipeinlagen aber ungeeignet für den Schutz von Tangaslips oder Stringtangaslips. Werden die gebräuchlichen Slipeinlagen für Tangaslips oder Stringtangaslips verwendet, so stehen zumindest Teile der Slipeinlage aus dem Slip hervor und die Klebeschicht kann in unangenehmer Weise mit dem Körper in Kontakt kommen.

Demgegenüber liegt der Erfindung das technische Problem zugrunde, eine Slipeinlage der eingangs genannten Art anzugeben, die auch für die Verwendung in Tangas und/oder Stringtangas geeignet ist.

Zur Lösung dieses technischen Problems lehrt die Erfindung eine Slipeinlage der eingangs genannten Art, welche dadurch gekennzeichnet ist, daß entlang einer von einer Längsseite zur anderen Längsseite der Slipeinlage verlaufenden Diagonalen eine Trennfuge in der Saugauflage vorgesehen ist
und daß die Saugauflage entlang der Diagonalen in zwei Teilsaugauflagen auftrennbar ist. Es liegt im Rahmen der Erfindung, daß jede der beiden Teilsaugauflagen eine entsprechende körperabgewandte Klebeschicht aufweist. Eine solche Teilsaugauflage kann dann mittels der körperabgewandten Klebeschicht in Tangas und/oder Stringtangas in entsprechender Weise eingelegt und festgeklebt werden. Vorzugsweise ist die Saugauflage entlang der Diagonalen in zwei Teilsaugauflagen gleicher Größe oder im wesentlichen gleicher Größe auftrennbar. Zweckmäßigerweise sind die beiden Teilsaugauflagen dreieckförmig mit jeweils zumindest zwei abgerundeten Dreieckseiten ausgeführt.

Längsseiten der Slipeinlage meint die beiden langen Seitenkanten in Längsrichtung der Slipeinlage. Es liegt im Rahmen der Erfindung, daß die Längsseiten über abgerundete Ecken in die Breitseiten der Slipeinlage übergehen. Insoweit kann die Diagonale auch im Bereich der abgerundeten Ecken beginnen oder enden. Diagonale meint im Rahmen der Erfindung eine Gerade oder eine im wesentlichen gerade Linie, wobei geringfügige Abweichungen von der Linearität möglich sind. Insbesondere kann die Diagonale auch als Zackenlinie oder Wellenlinie mit jeweils im Verhältnis zur Slipeinlagenoberfläche geringer Amplitude ausgeführt sein.

Nach bevorzugter Ausführungsform der Erfindung ist eine durchlaufende Trennfuge entlang der Diagonalen in der Saugauflage vorgesehen. Trennfuge meint im Rahmen der Erfindung aber auch entlang der Diagonalen ausgebildete Trennfugenabschnitte die durch Verbindungsabschnitte zwischen den beiden an die Trennfuge angrenzenden Teilsaugauflagen getrennt werden. In jeden Fall muß die Trennfuge ein einfaches Auftrennen der Saugauflage in die beiden Teilsaugauflagen per Hand gestatten. Insoweit liegt es auch im Rahmen der Erfindung, in der Saugauflage entlang der Diagonalen Lochstanzungen vorzusehen, die ein einfaches Auftrennen der Saugauflage in die zwei Teilsaugauflagen ermöglichen. Vorzugsweise liegen die durch die Trennfuge getrennten Teilsaugauflagen im Bereich der Trennfuge formschlüssig aneinander. Formschlüssiges Anliegen der beiden Teilsaugauflagen meint hier insbesondere, daß im ungeteilten Zustand der beiden Teilsaugauflagen keine Durchlaßöffnung im Bereich der Trennfuge ausgebildet ist. Mit anderen Worten kann die Slipeinlage auch als herkömmliche Slipeinlage für übliche Slips verwendet werden, ohne daß Flüssigkeit in nennenswertem Umfang durch die Trennfuge dringen kann. Zweckmäßigerweise liegen die beiden Teilsaugauflagen im Bereich der Trennfuge mit der Maßgabe formschlüssig aneinander, daß durch die Trennfuge nicht mehr oder nicht wesentlich mehr Flüssigkeit dringen kann als durch die übrigen Bereiche der Saugauflage. Grundsätzlich ist es aber auch möglich, daß die beiden Teilsaugauflagen im Bereich der Trennfuge einen geringen Abstand voneinander aufweisen. Dieser Abstand beträgt beispielsweise ein bis zwei Millimeter. - Vorzugsweise weisen die der Trennfuge zugewandten Kanten der Saugauflage eine Abschlußnaht auf.

Daß die Saugauflage gemäß Schutzanspruch 1 entlang der Diagonalen in zwei Teilsaugauflagen auftrennbar ist, meint im Rahmen der Erfindung insbesondere, daß die Saugauflage per Hand in die beiden Teilsaugauflagen auftrennbar bzw. auseinanderreißbar ist. Es liegt also im Rahmen der Erfindung, daß hierzu keinerlei Werkzeug, wie beispielsweise ein Messer oder eine Schere benötigt wird.

Zweckmäßigerweise weist die Saugauflage zumindest einen Vliesstoff auf. Die Saugauflage mag an ihrer körperabgewandten Fläche mit einer Folie, vorzugsweise Kunststoffolie, versehen sein, auf welcher Folie die Klebeschicht aufgebracht ist. Es versteht sich, daß dann auch die Folie als Bestandteil der Saugauflage entlang der Diagonalen auftrennbar ist. Zweckmäßigerweise weist auch die Folie die Trennfuge auf oder weist die Folie entlang der Diagonalen eine Reißperforation auf. Es liegt fernerhin im Rahmen der Erfindung, daß die Saugauflage körperseitig eine Abdeckschicht aus durchlässigem und/oder perforiertem Textilmaterial und/oder Kunststoffmaterial aufweist.

Nach bevorzugter Ausführungsform der Erfindung ist die Klebeschicht als vollflächige Klebeschicht auf der körperabgewandten Fläche der Slipeinlage aufgebracht. Es liegt jedoch auch im Rahmen der Erfindung, daß die Klebeschicht lediglich bereichsweise aufgebracht ist, beispielsweise in Form von in Längsrichtung der Slipeinlage parallelen Klebestreifen. - Nach einer weiteren bevorzugten Ausführungsform, der im Rahmen der Erfindung besondere Bedeutung zukommt, ist entlang der Diagonalen bzw. entlang der Trennfuge in der Klebeschicht eine klebeschichtfreie Zone vorgesehen.

Der auf der Klebeschicht aufgebrachte Schutzstreifen besteht vorzugsweise aus einer Kunststoffolie oder aus einer Papierfolie. Nach einer Ausführungsform der Erfindung ist der Schutzstreifen einteilig und nicht auftrennbar ausgebildet. Gemäß dieser Ausführungsform der Erfindung kann beispielsweise lediglich eine Teilsaugauflage von dem Schutzstreifen entfernt werden und die zweite Teilsaugauflage kann zunächst auf dem Schutzstreifen verbleiben. Nach einer weiteren Ausführungsform der Erfindung ist der Schutzstreifen entlang der Diagonalen in zwei Teilschutzstreifen auftrennbar und ist die Slipeinlage entlang der Diagonalen in zwei Teilslipeinlagen auftrennbar. Teilslipeinlage meint hier ein Aggregat aus der körperseitigen Teilsaugauflage mit körperabgewandter Klebeschicht und auf der Klebeschicht aufgebrachtem Teilschutzstreifen. Daß der Schutzstreifen entlang der Diagonalen in zwei Teilschutzstreifen auftrennbar ist meint im Rahmen der Erfindung insbesondere, daß der Schutzstreifen per Hand in die Teilschutzstreifen auftrennbar bzw. auseinanderreißbar ist. Insoweit liegt es im Rahmen der Erfindung, daß der Schutzstreifen entlang der Diagonalen eine Reißperforation aufweist und der Schutzstreifen aufgrund dieser Reißperforation in die beiden Teilschutzstreifen auftrennbar ist. Es versteht sich, daß die Reißperforation in dem Schutzstreifen so eingerichtet ist, daß ein einfaches, funktionssicheres und wenig kraftaufwendiges Trennen der Slipeinlage in die beiden Teilslipeinlagen möglich ist. Vorzugsweise ist entlang der Diagonalen bzw. entlang der Trennfuge eine Lochperforation in dem Schutzstreifen vorgesehen, wobei die einzelnen Löcher bevorzugt eng benachbart sind und beispielsweise einen Abstand von ein bis zwei Millimetern aufweisen. Reißperforation meint im Rahmen der Erfindung aber nicht zwingend, daß den Schutzstreifen durchgreifende Öffnungen entlang der Diagonalen vorgesehen sind. Mit anderen Worten kann in den Schutzstreifen auch eine Stanzkante oder Stanzrille entlang der Diagonalen eingebracht sein, die zweckmäßigerweise eine Auftrennung des Schutzstreifens in zwei Teilschutzstreifen ermöglicht. - Grundsätzlich liegt es auch im Rahmen der Erfindung, daß der Schutzstreifen von vornherein zweiteilig, d. h. in Form von zwei Teilschutzstreifen ausgebildet ist. Es versteht sich, daß in diesem Fall eine durchgehende Trennfuge in der Saugauflage nicht zweckmäßig ist. Bei einer solchen Ausführungsform sind die beiden Teilsaugauflagen vor dem Auftrennen über Befestigungselemente oder Befestigungsabschnitte miteinander verbunden. Bei der zweiteiligen Ausführungsform des Schutzstreifens können sich die beiden Teilschutzstreifen im Bereich der Diagonalen überlappen.

Nach bevorzugter Ausführungsform der Erfindung sind die Teilslipeinlagen dreieckförmig mit jeweils zumindest zwei abgerundeten Dreiecksecken ausgeführt. Zweckmäßigerweise ist die Slipeinlage in zwei Teilslipeinlagen von im wesentlichen gleicher Größe auftrennbar.

Nach bevorzugter Ausführungsform der im Rahmen der Erfindung besondere Bedeutung zukommt, ist zumindest die Saugauflage der Slipeinlage gefärbt. Dabei liegt es im Rahmen der Erfindung, daß die Saugauflage in Modefarben eingefärbt ist, die den Modefarben der Tangas oder Stringtangas entsprechen. Vorzugsweise ist auch die körperabgewandte slipseitige Fläche der Slipeinlage entsprechend eingefärbt. Durch die erfindungsgemäße Einfärbung der Slipeinlagen wird erreicht, daß sich die Slipeinlagen bzw. die Teilslipeinlagen nicht in unerwünschter und unästhetischer Weise kontrastbildend in der Unterwäsche abzeichnen.

Es liegt auch im Rahmen der Erfindung, daß die beiden Teilsaugauflagen der Slipeinlage im Bereich der Trennfuge bzw. im Bereich der Diagonalen einander überlappend ausgeführt sind. Bei dieser Ausführungsform kann die Trennfuge als durchgehende Trennfuge ohne Verbindungsabschnitte zwischen den beiden Teilsaugauflagen ausgebildet sein oder es können auch Verbindungsabschnitte zwischen den beiden Teilsaugauflagen vorhanden sein. Wenn bei dieser Ausführungsform die Saugauflage an ihrer körperabgewandten Fläche eine Folie aufweist, auf welcher Folie vorzugsweise die Klebeschicht aufgebracht ist, kann diese Folie im Bereich der Trennfuge bzw. im Bereich der Diagonalen so eingerichtet sein, daß sie auf einfache Weise per Hand entlang der Diagonalen in zwei Teilfolien auftrennbar ist. So kann die Folie beispielsweise eine entsprechende Perforation entlang der Diagonalen aufweisen. Bei dieser Ausführungsform mit einander überlappenden Teilsaugauflagen kann der Schutzstreifen entweder einteilig und nicht auftrennbar ausgebildet sein oder der Schutzstreifen ist so eingerichtet, daß er auf einfache Weise per Hand entlang der Diagonalen in zwei Teilschutzstreifen auftrennbar ist. Die Ausbildungsmöglichkeiten des Schutzstreifens im Hinblick auf eine leichte Auftrennbarkeit in zwei Teilschutzstreifen wurden weiter oben bereits erläutert.

Der Erfindung liegt die Erkenntnis zugrunde, daß gebräuchliche und übliche Slipeinlagen auf einfache und wenig aufwendige Weise auch zu Slipeinlagen für Tangas oder Stringtangas umfunktioniert werden können, wenn die erfindungsgemäßen Merkmale verwirklicht werden. In herstellungstechnischer Hinsicht bereitet die Einarbeitung einer Trennfuge in die Saugauflage herkömmlicher Slipeinlagen keinerlei Probleme. Der maschinelle bzw. apparative Aufwand hierfür ist gering. Besondere Bedeutung kommt im Rahmen der Erfindung der Tatsache zu, daß die erfindungsgemäß eingerichteten Slipeinlagen nichtsdestoweniger funktionssicher und ohne Beeinträchtigung ihrer Wirkungsweise als herkömmliche Slipeinlagen eingesetzt werden können, wenn die Auftrennung in die zwei Teilslipeinlagen nicht vorgenommen wird. Die erfindungsgemäß aus der Slipeinlage hergestellten Teilslipeinlagen passen sich in hervorragender Weise in übliche Tangas oder Stringtangas ein. Sie zeichnen sich hier trotz ihrer verhältnismäßig geringen Größe durch eine überraschend hohe Wirksamkeit bzw. Funktionssicherheit aus. Mit anderen Worten können mit den erfindungsgemäß erhaltenen Teilslipeinlagen die Tangas oder Stringtangas wirkungsvoll geschützt werden.

Nachfolgend wird die Erfindung anhand einer lediglich ein Ausführungsbeispiel darstellenden Zeichnung näher erläutert. Es zeigen in schematischer Darstellung:
- Fig. 1: eine erste Ausführungsform der erfindungsgemäßen Slipeinlage in der Draufsicht,
- Fig. 2: eine Rückansicht auf den Schutzstreifen des Gegenstandes gemäß Fig. 1,
- Fig. 3: den Gegenstand nach Fig. 1, aufgetrennt in zwei Slipeinlagen und
- Fig. 4: eine andere Ausführungsform des Gegenstandes nach Fig. 1.

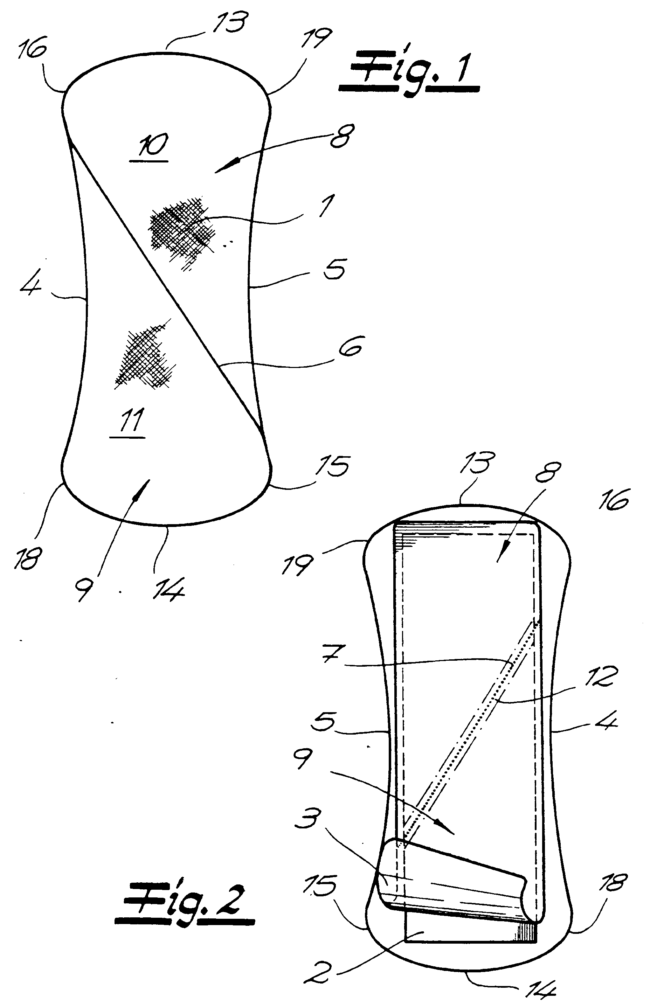

Die Figuren zeigen eine Slipeinlage mit körperseitiger Saugauflage 1, körperabgewandter Klebeschicht 2 und auf der Klebeschicht 2 aufgebrachtem Schutzstreifen 3. Die Fig. 1 zeigt eine herkömmliche Form einer Slipeinlage mit konkav zur Slipeinlagenmitte ausgerichteten Längsseiten 4, 5 sowie konvex ausgebildeten Breitseiten 13, 14. Entlang einer von einer Längsseite 4 zur anderen Längsseite 5 der Slipeinlage verlaufenden Diagonalen ist eine Trennfuge 6 in der Saugauflage 1 vorgesehen. Es liegt auch im Rahmen der Erfindung, daß die Diagonale bzw. die Trennfuge 6 im Bereich der abgerundeten Ecken 15, 16 der Längsseiten 4, 5 beginnt bzw. endet. Der Begriff Längsseite umfaßt im Rahmen der Erfindung also auch diese abgerundeten Ecken 15, 16. Nach bevorzugter Ausführungsform und im Ausführungsbeispiel ist die Trennfuge 6 als durchlaufende Trennfuge 6 entlang der Diagonalen vorgesehen. Vorzugsweise und im Ausführungsbeispiel liegen die durch die Trennfuge 6 getrennten Teilsaugauflagen 10, 11 im Bereich der Trennfuge 6 formschlüssig aneinander. Mit anderen Worten stoßen die beiden Teilsaugauflagen 10, 11 mit ihren Stoßkanten aneinander. Nach sehr bevorzugter Ausführungsform der Erfindung und im Ausführungsbeispiel liegen die Teilsaugauflagen 10, 11 mit der Maßgabe formschlüssig aneinander, daß im unaufgetrennten Zustand der Saugauflage 1 eine Dichtfunktion der Trennfuge gegen das Durchdringen von Flüssigkeit gewährleistet ist. Insoweit liegt es im Rahmen der Erfindung, daß im unaufgetrennten Zustand der Saugauflage 1 keine Zwischenräume im Bereich der Trennfuge zwischen den Teilsaugauflagen 10, 11 ausgebildet sind, die ein Durchdringen von Flüssigkeit ermöglichen würden.

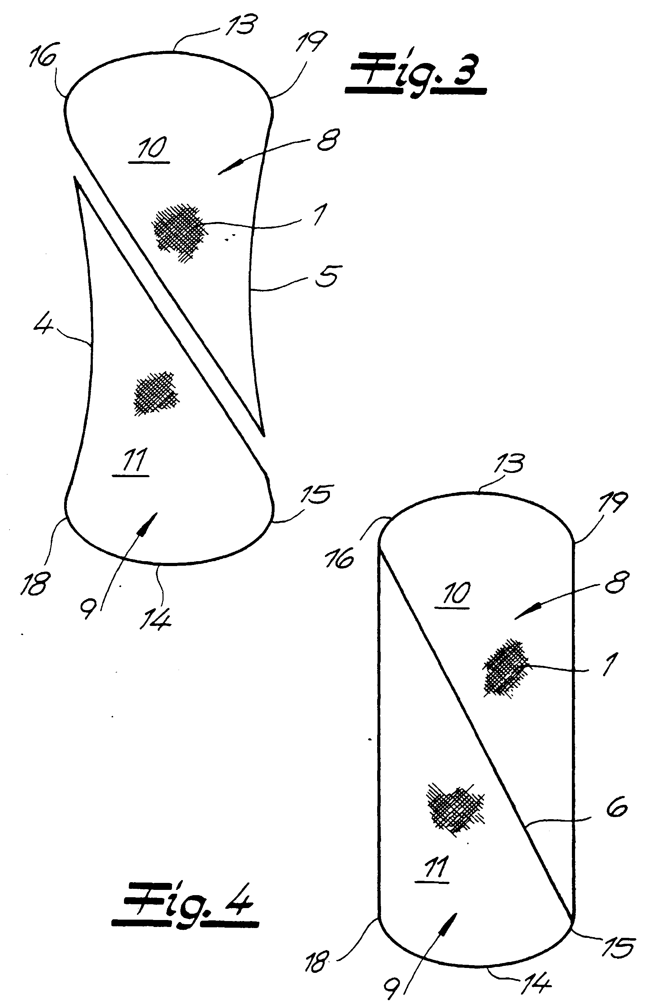

Der Schutzstreifen 3 weist entlang der Diagonalen eine Reißperforation 7 auf (Fig. 2). Die Reißperforation ist im Ausführungsbeispiel als linienförmige Lochperforation entlang der Diagonalen ausgeführt. In Fig. 2 ist der Schutzstreifen 3 bereichsweise angehoben bzw. abgezogen dargestellt. Unterhalb des Schutzstreifens 3 befindet sich die Klebeschicht 2, die vorzugsweise und im Ausführungsbeispiel vollflächig auf der Rückseite der Saugauflage 1 aufgebracht ist. Die Klebeschicht 2 kann auf eine an der körperabgewandten Seite der Saugauflage 1 befestigte, in den Figuren nicht dargestellte Kunststoffolie aufgebracht sein. Zweckmäßigerweise weist dann auch die Kunststotfolie entlang der Diagonalen die Trennfuge 6 auf. Die Kunststoffolie ist aber nicht zwingend erforderlich. - Vorzugsweise ist entlang der Trennfuge 6 bzw. entlang der Diagonalen in der Klebeschicht 2 eine klebeschichtfreie Zone 12 vorgesehen. Diese klebeschichtfreie Zone 12 ist in Fig. 2 strichpunktiert angedeutet worden.

Die Slipeinlage ist aufgrund der Trennfuge 6 in der Saugauflage 1 sowie der Reißperforation 7 in dem Schutzstreifen 3 entlang der Diagonalen in zwei Teilslipeinlagen 8, 9 auftrennbar. Fig. 3 zeigt die beiden Teilslipeinlagen 8, 9 nach dem Auftrennen der Slipeinlage. Vorzugsweise und im Ausführungsbeispiel sind die Teilslipeinlagen 8, 9 dreieckförmig mit zwei abgerundeten Dreiecksecken. 15, 18 bzw. 16, 19 ausgeführt. Es versteht sich, daß die Seiten bzw. Kanten der dreieckförmigen Teilslipeinlagen 8, 9 in Abhängigkeit von der Form der ursprünglichen Slipeinlage ausgebildet sind. Mit anderen Worten weisen die Teilslipeinlagen 8, 9 in der Regel keine geraden Dreiecksseiten auf. Nach bevorzugter Ausführungsform und im Ausführungsbeispiel weisen die beiden Teilslipeinlagen 8, 9 die gleiche Größe auf.

Fig. 4 zeigt eine andere Ausführungsform einer Slipeinlage, welche in zwei Teilslipeinlagen 8, 9 auftrennbar ist, die gegenüber den Teilslipeinlagen 8, 9 in Fig. 3 eine größere Längserstreckung aufweisen. Sowohl die Teilslipeinlagen 8, 9 nach den Fig. 1 bis 3 als auch die Teilslipeinlagen 8, 9 nach Fig. 4 lassen sich funktionssicher in Tangas bzw. Stringtangas einpassen bzw. mit ihrer jeweiligen Klebeschicht in den Tangas bzw. Stringtangas festkleben.

## Patentansprüche

1. Slipeinlage, - mit
körperseitiger Saugauflage (1), körperabgewandter Klebeschicht (2) und auf der Klebeschicht (2) aufgebrachtem Schutzstreifen (3),
**dadurch gekennzeichnet**, daß entlang einer von einer Längsseite (4) zur anderen Längsseite (5) der Slipeinlage verlaufenden Diagonalen eine Trennfuge (6) in der Saugauflage (1) vorgesehen ist und daß die Saugauflage (1) entlang der Diagonalen in zwei Teilsaugauflagen (10, 11) auftrennbar ist.

2. Slipeinlage nach Anspruch 1, dadurch gekennzeichnet, daß eine durchlaufende Trennfuge (6) entlang der Diagonalen vorgesehen ist.

3. Slipeinlage nach einem der Ansprüche 1 oder 2, dadurch gekennzeichnet, daß die durch die Trennfuge (6) getrennten Teilsaugauflagen (10, 11) im Bereich der Trennfuge (6) formschlüssig aneinander liegen.

4. Slipeinlage nach einem der Ansprüche 1 bis 3, dadurch gekennzeichnet, daß entlang der Trennfuge (6) in der Klebeschicht (2) eine klebeschichtfreie Zone (12) vorgesehen ist.

5. Slipeinlage nach einem der Ansprüche 1 bis 4, dadurch gekennzeichnet, daß der Schutzstreifen (3) entlang der Diagonalen in zwei Teilschutzstreifen auftrennbar ist und daß die Slipeinlage entlang der Diagonalen in zwei Teilslipeinlagen (8, 9) auftrennbar ist.

6. Slipeinlage nach Anspruch 5, dadurch gekennzeichnet, daß die Teilslipeinlagen (8, 9) dreieckförmig mit jeweils zumindest zwei abgerundeten Dreiecksecken ausgeführt sind.

7. Slipeinlage nach einem der Ansprüche 5 oder 6, dadurch gekennzeichnet, daß die Slipeinlage in zwei Teilslipeinlagen (8, 9) gleicher Größe auftrennbar ist.
